# FASCICULE DE BREVET EUROPEEN

(11) **EP 2 731 585 B1**
(45) Date de publication et mention de la délivrance du brevet: **22.03.2017**
(21) Numéro de dépôt: 12743515.4
(22) Date de dépôt: 12.07.2012
(51) Int. Cl.: A61K 8/60, A61K 31/7016, A61L 15/16, A61L 15/22, A61L 15/44, A61Q 19/00, A61P 17/02, A61K 8/73, A61Q 19/06, A61K 45/06

(54) **UTILISATION COSMETIQUE ET/OU DERMATOLOGIQUE DE COMPOSES OLIGOSACCHARIDIQUES POUR LA PREVENTION ET LE TRAITEMENT DES VERGETURES**
VERWENDUNG VON OLIGOSACCHARIDEN ZUR PRÄVENTION UND BEHANDLUNG VON PATHOLOGISCHEN NARBEN
USE OF OLIGOSACCHARIDE COMPOUNDS FOR THE PREVENTION AND TREATMENT OF PATHOLOGICAL SCARS

(30) Priorité: 13.07.2011 FR 1156431
(43) Date de publication de la demande: 21.05.2014
(73) Titulaire: URGO RECHERCHE INNOVATION ET DEVELOPPEMENT, 21300 Chenôve (FR)
(72) Inventeur: APERT, Laurent, 21000 Dijon (FR); BOUSCHBACHER, Marielle, 21220 Chambolle-Musigny (FR)
(74) Mandataire: Cabinet Plasseraud
(86) Numéro de dépôt international: PCT/FR2012/051669
(87) Numéro de publication internationale: WO 2013/007961

(56) Documents cités:
- EP-A2- 0 415 458
- WO-A1-02/089818
- WO-A2-01/17486
- BR-A- PI0 902 206
- FR-A1- 2 784 029
- FR-A1- 2 824 474
- FR-A1- 2 916 355
- FR-A1- 2 953 522

## Description

La présente invention concerne une composition topique, notamment cosmétique et/ou dermatologique, utile pour prévenir et/ou traiter les vergetures, comprenant, dans un support physiologiquement acceptable, une quantité efficace d'au moins un oligosaccharide polysulfaté synthétique ayant 1 à 4 unités oses, ses sels et/ou dérivés.

Les vergetures touchent près de 50% de la population jeune, essentiellement féminine et malgré une régression spontanée partielle, elles laissent des cicatrices définitives qui vieillissent mal.

Cliniquement, les vergetures se présentent comme des stries linéaires ou fusiformes en « coups de verge » de 1/2cm à plusieurs centimètres de longueur sur 1 mm à 1 cm de largeur, symétriques et multiples. Leur surface peut être lisse et tendue, lorsqu'elles sont récentes au stade inflammatoire, ou fripée, déprimée, avec une impression de vacuité au toucher et un aspect de peau en papier à cigarettes froissé, lorsqu'elles sont anciennes. Glabres sans sécrétion sudorales ni sébacée, leur couleur évolue du rose-rouge-pourpre au blanc nacrée. Elles apparaissent plus fréquemment chez la femme que chez l'homme, et sont localisées sur l'abdomen, les fesses, les hanches, les racines des membres, les seins, ou les épaules.

Elles sont le résultat d'une distension anormale de la peau survenant au cours de certaines affections ou épisodes de la vie comme la grossesse ou un surpoids important. Les vergetures résultent d'une rupture des fibres de collagène et d'élastine dans la couche profonde du derme sans qu'aucune lésion de l'épiderme soit observée. Les vergetures se distinguent des cicatrices « usuelles » qui sont issues de plaies ouvertes, de brûlures ou d'une pathologie puisque ces plaies résultent d'une lésion de l'épiderme et d'une partie plus ou moins profonde du derme. La peau d'un individu obèse, par exemple, est soumise à une distension en rapport direct avec la quantité de graisse mise en réserve. Après amaigrissement, la peau ne retrouve pas sa structure habituelle et reste étirée, distendue. C'est la raison pour laquelle chez certains obèses, des vergetures peuvent apparaitre. Elles peuvent également se former au niveau des membres lorsque la croissance osseuse a été particulièrement rapide. Elles sont également la conséquence de l'utilisation inadaptée de corticoïdes (cortisone), soit sous forme de pommade, soit, le plus souvent, sous forme injectable. Les vergetures sont également le résultat d'une sécrétion exagérée d'hormones corticoïdiennes (maladie de Cushing). Il peut s'agir de cicatrices de forme arrondie survenant au cours de la syphilis secondaire ou de la typhoïde. Les haltérophiles sont également susceptibles de présenter des vergetures : c'est le cas d'ailleurs d'un certain nombre de sportifs.

Aujourd'hui, il n'existe pas réellement de traitement efficace pour lutter contre les vergetures. Les massages, les dérivés d'extrait de placenta, les crèmes au collagène, les crèmes à l'élastine, les dérivés de silicium n'ont pas démontré leur efficacité. Certains sujets peuvent parfois avoir recours à la chirurgie pour résorber la quantité trop importante de peau (tablier excessif), mais cette intervention ne s'applique pas aux vergetures elles-mêmes.

Il existe donc un besoin constant de trouver une solution efficace pour le traitement de ce type de cicatrices.

Ainsi, le brevet européen EP-B-1 784 205 décrit l'utilisation d'une toxine botulique pour la fabrication d'un médicament pour traiter une vergeture où ladite toxine botulinique est destinée à être administrée à une vergeture ou au voisinage d'une vergeture d'un patient. Cependant l'utilisation d'une toxine botulique est déconseillée chez certains sujets, par exemple les femmes enceintes ou les sujets sous traitement antibiotique par les aminosides (Amiklin, Gentalline ou Streptomycine par exemple), sous traitement anticoagulant récent, sous traitement anti-inflammatoire récent, ou présentant une hypersensibilité connue au botox®, une myasthénie grave, le syndrôme de Lambert-Eaton. De plus l'injection, qui reste un geste chirurgical nécessitant du personnel qualifié, peut présenter des risques de contamination.

Le brevet WO 02102348 concerne un produit anti-vergetures comprenant une préparation destinée à traiter les vergetures de la peau, dans un format de bâtonnet, et un distributeur recevant cette préparation et comprenant un moyen de distribution pouvant être manipulé par un utilisateur et permettant la répartition de la préparation sur une extension recherchée. Cependant la composition est complexe ainsi que son mode d'application.

Enfin, le brevet français FR2857596 revendique une composition topique particulière pour le traitement préventif des vergetures adaptée pour les femmes enceintes, et contenant un extrait riche de lupéol.

Il existe un besoin aujourd'hui pour une nouvelle composition cosmétique ayant une action sur les vergetures qui soit efficace.

De manière inattendue, la demanderesse a constaté que l'utilisation d'un composé oligosaccharide polysulfaté synthétique particulier permettait de traiter efficacement les vergetures.

L'invention a donc pour objet une composition cosmétique et/ou dermatologique, pour son utilisation pour prévenir et/ou traiter les vergetures comprenant, dans un support physiologiquement acceptable, au moins une quantité efficace d'au moins un d'oligosaccharide polysulfaté synthétique ayant 1 à 4 unités oses, ses sels et/ou dérivés.

Au sens de la présente invention, on entend par oligosaccharide polysulfaté synthétique, des polymères synthétiques formés de 1 à 4 unités d'oses, et de préférence de 1 ou 2 unités d'oses, généralement liées entre elles par liaison glycosidique alpha ou bêta. En d'autres termes, il s'agit de mono, di, tri ou tétrasaccharides, et de préférence de mono ou disaccharides.

Selon un autre de ses aspects, l'invention a pour objet un procédé cosmétique comprenant au moins une étape d'application sur la peau d'une composition topique comprenant au moins une quantité efficace d'au moins un oligosaccharide polysulfaté synthétique ayant de 1 à 4 unités d'oses, ses sels et/ou dérivés.

Selon encore un autre de ses aspects, l'invention a pour objet l'utilisation d'une quantité efficace d'au moins un oligosaccharide polysulfaté synthétique ayant de 1 à 4 unités d'oses, ses sels et/ou dérivés, en association avec une quantité efficace d'au moins une substance active, pour la fabrication d'une composition cosmétique ou dermatologique destinée à traiter ou prévenir les vergetures.

On entend par "composition cosmétique et/ou dermatologique"au sens de la présente demande, une composition pour application topique, comportant un milieu cosmétiquement ou dermatologiquement acceptable, c'est-à-dire qui présente une couleur, une odeur et un toucher agréables et qui ne génère pas d'inconforts inacceptables (picotements, tiraillements, rougeurs), susceptibles de détourner les consommateurs de l'utilisation de cette composition.

Par "quantité efficace", on entend au sens de la présente invention une quantité suffisante pour obtenir l'effet attendu.

Par "support physiologiquement acceptable", on désigne un support non toxique et susceptible d'être appliqué sur au moins une matière kératinique d'êtres humains.

### Oligosaccharide polysulfaté synthétique

La composition selon l'invention comprend au moins un composé d'oligosaccharide polysulfaté synthétique ayant de 1 à 4 unités d'oses, ses sels et/ou dérivés.

Les oligosaccharides utilisés dans le cadre de la présente invention sont des oligomères synthétiques formés de 1 à 4 unités d'oses, et de préférence de 1 ou 2 unités d'oses, généralement liées entre elles par liaison glycosidique alpha ou bêta. En d'autres termes, il s'agit de mono, di, tri ou tétrasaccharides, et de préférence de mono ou disaccharides.

Il n'y a pas de limitation particulière concernant la nature des unités oses de ces polysaccharides. De préférence, il s'agira de pentoses ou d'hexoses. A titre d'exemple de monosaccharide, on peut citer le glucose, le galactose ou le mannose. A titre d'exemple de disaccharide, on peut citer le maltose, le lactose, le sucrose ou le tréhalose. A titre d'exemple de trisaccharide, on peut citer le mélézitose. A titre d'exemple de tétrasaccharide, on peut citer le stachyose.

De préférence, l'oligosaccharide est un disaccharide, et de préférence encore le sucrose.

On entend par "oligosaccharide polysulfaté" au sens de la présente demande un oligosaccharide dont au moins deux, et de préférence tous les groupes hydroxyles de chaque ose ont été substitués par un groupe sulfate.

De préférence, l'oligosaccharide polysulfaté utilisé dans le cadre de la présente demande est le sucrose octasulfate.

Les oligosaccharides polysulfatés utilisés dans le cadre de la présente invention peuvent se présenter sous forme de sels ou complexes.

A titre d'exemple de sels, on peut citer les sels de métal alcalin tels que les sels de sodium, de calcium ou de potassium, les sels d'argent ; ou encore les sels d'acide aminé.

A titre d'exemple de complexes, on peut citer les complexes d'hydroxyaluminium.

Dans le cadre de la présente invention, des composés particulièrement préférés sont les suivants :
- le sel de potassium du sucrose octasulfaté (KSOS) ;
- le sel d'argent du sucrose octasulfaté ;
- le complexe hydroxyaluminium du sucrose octasulfate, appelé communément sucralfate.

En particulier, dans le cadre de la présente invention, les oligosaccharides polysulfatés utilisés sont de préférence les sels de potassium plutôt que les sels d'aluminium du sucrose octasulfaté.

Les oligosaccharides polysulfatés utilisés dans le cadre de la présente invention peuvent se présenter sous forme de poudre micronisée ou sous forme solubilisée.

De préférence, l'oligosaccharide polysulfaté préféré dans le cadre de la présente invention est le sel de potassium du sucrose octasulfate (connu sous l'abréviation KSOS), commercialisé par exemple dans le produit Urgotul® Start en tant que « NOSF » par les Laboratoires URGO®.

En particulier, le composé d'oligosaccharide polysulfaté synthétique est présent dans la composition selon l'invention en une teneur comprise entre 0,1 et 50% en poids, par rapport au poids total de la composition.

Les composés oligosaccharides polysulfatés synthétiques peuvent être introduits, dans les compositions selon l'invention, seuls ou en mélange de deux ou plus d'entre eux, ou encore en combinaison avec une (ou plusieurs) autre(s) substance(s).

Selon un mode particulier de réalisation de l'invention, la composition comprend au moins une quantité d'oligosaccharides polysulfatés synthétiques comprise entre 0,1 et 50% en poids, par rapport au poids total de la composition.

Selon un mode particulier de réalisation de l'invention, la composition comprend au moins une quantité d'oligosaccharides polysulfatés synthétiques telle que la quantité relarguée sur la vergeture est comprise entre 0,001 g/l et 50 g/l, et de préférence encore entre 0,01 et 10 g/l.

### Substance active

Outre les composés oligosaccharides polysulfatés synthétiques, la composition selon l'invention peuvent comprendre une (ou plusieurs) autre(s) substance(s) active(s).

De manière générale, les actifs sont choisis parmi les anti-bactériens, les antiseptiques, les antifongiques, les anti-inflammatoires, les actifs favorisant la cicatrisation et/ou la restructuration de la peau, les antiprurigineux, les filtres UV, les agents apaisants, les actifs hydratants, les agents dépigmentants, les agents kératolytiques, les anti-viraux, les anti-douleurs, les anesthésiques, les vitamines et leurs mélanges.

De manière générale, les actifs sont choisis parmi :
- les anti-bactériens tels que le Polymine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol, les sels d'argent ;
- les anti fongiques tels que les polyènes, le Nystatin, l'Amphotéricine B, la Natamycine, les imidazolés (Miconazole, Ketoconazole, Clotrimazole, Éconazole, Bifonazole, Butoconazole, Fenticonazole, Isoconazole, Oxiconazole, Sertaconazole, Sulconazole, Thiabendazole, Tioconazole), les triazolés (Fluconazole, Itraconazole, Ravuconazole, Posaconazole, Voriconazole), les allylamines, la Terbinafine, l'Amorolfine, la Naftifine, la Buténafine ;
- la Flucytosine (antimétabolite), la Griséofulvine, la Caspofungine, la Micafungine, l'arginine ;
- les anti-inflammatoires tels que les Glucocorticoïdes, les anti-inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les actifs favorisant la cicatrisation et/ou la restructuration de la peau tels que le Rétinol, les vitamines hydrosolubles telles que la Vitamine A ou ses dérivés, la Vitamine E ou ses dérivés, la N-acétyl-hydroxyproline, les extraits de *Centella Asiatica* et d'aneth, la papaïne, les silicones, les huiles essentielles de thym, de niaouli, de romarin et de sauge, l'acide hyaluronique, l'allantoïne, Héma'tîte^{®} (Gattefossé), Vitamine C, TEGO^{®} Pep 4-17 (Evonik), Toniskin^{®} (Silab), Collageneer^{®} (Expanscience), Timecode^{®} (Seppic), Gatuline^{®} skin repair (Gattefossé), Panthenol, PhytoCellTec^{®} Alp Rose (MibelleBiochemistry), Serilesine^{®} (Lipotec), Hétérosides de Talapetraka (Bayer), Stoechiol^{®} (Codif), macarose (Sensient), Dermaveil (Ichimaru Pharcos), Phycosaccharide AI (Codif), la metformine ;
- les agents dépigmentants tels que l'acide kojique (KojicAcid SL^{®} - Quimasso (Sino Lion)), l'Arbutine (Olevatin^{®} - Quimasso (Sino Lion)), le mélange de palmitoylpropyl de sodium et d'extrait de nénuphar blanc (Sepicalm^{®} - Seppic), l'undécylénoyl phénylalanine (Sepiwhite^{®} - Seppic), l'extrait de réglisse obtenue par fermentation d'*Aspergillus* et éthoxydiglycol (GatulineWhitening^{®} - Gattefossé), l'acide octadécènedioïque (ODA White^{®} - Sederma), l'alpha-arbutin (Alpha-arbutin^{®}, SACI-CFPA (Pentapharm)), l'extrait aqueux de feuilles *Arctophylos Uva Ursi* (Melfade-J^{®} - SACI-CFPA (Pentapharm)), le mélange de plante complexe Gigawhite^{®} (SACI-CFPA (Alpaflor)), la diacétylboldine (Lumiskin^{®} - Sederma), l'extrait de mandarine du Japon (Melaslow^{®} - Sederma), le mélange d'extrait de citron enrichi en acide citrique et d'extrait de concombre (Uninontan^{®}U-34 - Unipex), le mélange d'extrait de *Rumex occidentalis* et de vitamine C (Tyrostat^{®} 11 - Unipex), des oligopeptides (Mélanostatine 5^{®} - Unipex), le dipalmitatekojique (KAD-15^{®} - Quimasso (Sino Lion)), le complexe d'origine naturelle Vegewhite^{®} de LCW, des extraits de germe de blé (Clariskin^{®} II - Silab), l'acide éthylènediamine tétracétique (EDTA);
- Les antiprurigineux tels que l'hydrocotisone, l'enoxolone, la diphénhydramine, les antihistaminiques à application locale anti H1 ;
- Des filtres UV tels que Parsol^{®} MCX, Parsol^{®} 1789 ;
- Des agents apaisants tels que la camomille, le bisabolol, le Zanthalène^{®}, l'acide glycyrrhébénique, la Tanactine^{®} (CPN), la Calmiskin^{®} (Silab) ;
- Des actifs hydratants tels que le Xpermoist^{®} (Lipotec), l'acide hyaluronique, l'urée, les acides gras, la glycérine, les cires, Exossine^{®} (Unipex) ;
- les agents kératolytiques tels que l'acide salicylique, le salicylate de zinc, l'acide ascorbique, les acides alpha hydroxylés (acide glycolique, lactique, malique, citrique, tartrique), les extraits d'Erable argenté, de Griottier, de Tamarinier, l'urée, le rétinoïde topique Kératoline^{®} (Sederma), les protéases obtenues par fermentation de *Bacillus Subtilis*, le produit Linked-Papain^{®} (SACI-CFPA), la papaïne (enzyme protéolytique issue du fruit de papaye) ;
- Les vitamines telles que le rétinol (vitamine A) et ses dérivés, le tocophérol (vitamine E) et ses dérivés.

Selon un mode préféré de réalisation, la composition selon l'invention comprend au moins une quantité efficace d'un actif favorisant la cicatrisation et/ou la restructuration de la peau, de préférence l'acide hyaluronique.

On peut, en outre, associer les actifs selon l'invention, à des agents actifs destinés notamment à la prévention et/ou au traitement des affections cutanées.

### Galénique

Les compositions selon l'invention peuvent se présenter sous toutes les formes galéniques normalement utilisées par l'homme du métier.

Lorsque les compositions sont destinées à une administration topique, elles peuvent se présenter sous la forme de solutions (aqueuses, hydroalcooliques ou huileuses), de dispersions (type lotion ou sérum), d'émulsions de consistance liquide ou semi-liquide (type lait), de suspensions ou émulsions (type crème), de gel aqueux ou lipophile, de microémulsions, de microcapsules, de microparticules, ou de dispersions vésiculaires de type ionique et/ou non ionique. Elles peuvent également se présenter sous la forme de pansements interfaces non absorbants.

Ces compositions sont préparées selon les méthodes usuelles.

Elles peuvent notamment se présenter sous la forme de crèmes de traitement ou de soin pour le visage, pour les grands plis anatomiques ou pour le corps,

Les compositions selon l'invention peuvent également consister en des préparations solides telles que des savons ou des pains de nettoyage.

Bien entendu, la quantité d'oligosaccharides polysulfatés synthétiques utilisée dans la formulation galénique selon l'invention est adaptée en fonction de la cinétique recherchée ainsi que des contraintes spécifiques liées à sa nature, solubilité, résistance à la chaleur, etc.

D'une manière générale, lorsqu'il est utilisé dans une formulation galénique, le composé oligosaccharide polysulfaté synthétique selon l'invention pourra être incorporé en une teneur comprise entre 0,1 et 50% en poids, par rapport au poids total de la formulation.

### Eau et solvants hydrosolubles

Selon un mode préféré de réalisation, la composition de l'invention peut également comprendre de l'eau, et notamment une eau thermale et/ou minérale.

La composition selon l'invention peut également comprendre un ou plusieurs solvants miscibles à l'eau, tels que les alcools inférieurs, notamment l'éthanol et l'isopropanol, le propylène glycol.

### Phase grasse

La composition selon l'invention peut également comprendre une phase grasse.

Lorsque la composition de l'invention se présente sous la forme d'une émulsion, la proportion de la phase grasse peut aller de 5 à 80 % en poids, et de préférence de 5 à 50 % en poids, par rapport au poids total de la composition.

Lorsque la composition de l'invention est une solution ou un gel huileux, la phase grasse peut représenter plus de 90 % du poids total de la composition.

Les huiles utilisées dans la composition selon l'invention sont choisies parmi celles classiquement utilisées dans le domaine cosmétique et/ou dermatologique.

Comme matières grasses utilisables dans l'invention, on peut citer, outre les acides gras insaturés, les huiles minérales comme par exemple le polyisobutène hydrogéné et l'huile de vaseline, les huiles végétales comme par exemple une fraction liquide du beurre de karité, l'huile de tournesol, d'amande et d'abricot, les huiles animales comme par exemple le perhydrosqualène, les huiles de synthèse notamment l'huile de purcellin, le myristate d'isopropyle et le palmitate d'éthylhexyle, et les huiles fluorées comme par exemple les perfluoropolyéthers.

On peut aussi utiliser des alcools gras, des acides gras comme par exemple l'acide stéarique et des cires notamment de paraffine, carnauba et la cire d'abeille.

On peut aussi utiliser des composés siliconés comme les huiles siliconées et par exemple les cyclométhicones et diméthicones, les cires, les résines et les gommes siliconées.

### Emulsionnants

La composition selon l'invention, lorsqu'elle se présente sous forme d'émulsion, peut également comprendre un ou plusieurs émulsionnants et/ou co-émulsionnants.

Les émulsionnants et les co-émulsionnants utilisés dans la composition sont choisis parmi ceux classiquement utilisés dans le domaine cosmétique et/ou dermatologique.

Comme émulsionnant utilisable dans l'invention, on peut citer par exemple le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO® par la société HENKEL, le mélange de PEG-6/PEG-32/Glycol Stéarate vendu sous la dénomination de Tefose® 63 par société GATTEFOSSE, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (20OE).

La teneur totale en émulsionnants et co-émulsionnants peut notamment aller de 0,3 à 30 % en poids, et de préférence de 0,5 à 20 % en poids, par rapport au poids total de la composition.

### Gélifiants

La composition selon l'invention peut également comprendre des agents gélifiants.

Comme gélifiants hydrophiles, on peut citer les polymères carboxyliques tels que les carbomers, les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305® par la société SEPPIC, les polysaccharides comme les dérivés cellulosiques tels que les hydroxyalkylcelluloses et en particulier, les hydroxypropylcellulose et hydroxyéthylcellulose, les gommes naturelles telles que le guar, la caroube, la gomme de xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

### Adjuvants

La composition cosmétique et/ou dermatologique de l'invention peut contenir également des adjuvants habituels dans le domaine cosmétique, pharmaceutique et/ou dermatologique, tels que les conservateurs, les antioxydants, les solvants, les parfums, les charges, les absorbeurs d'odeur et les matières colorantes. Ces adjuvants, selon leur nature, peuvent être introduits dans la phase grasse et/ou dans la phase aqueuse de la composition.

Les quantités de ces différents adjuvants sont celles classiquement utilisées dans le domaine considéré, et par exemple de 0,01 à 20 % du poids total de la composition.

### Film

Selon un mode préféré de réalisation de l'invention, la composition se présente sous la forme d'un film, de préférence un film polymérique hydrosoluble de type filmogel®.

Par "film", on entend selon la présente invention, un solide fin.

On entend par "fin", un solide ayant une épaisseur d'au maximum 1000 µm

Ce film est préhensible, c'est-à-dire qu'il a généralement une dimension adéquate pour pouvoir être facilement manipulé par l'utilisateur. Il peut avoir une forme de carré, de rectangle, de disque ou toute autre forme. Un film a généralement une épaisseur de 10 µm à 1000 µm, de préférence de 20 à 500 µm et mieux de 50 à 300 µm.

On entend par "film hydrosoluble" au sens de la présente invention, un film qui se dissout dans l'eau. Il s'agit d'un film constitué d'un ou de plusieurs polymères hydrosolubles ou hydrodispersibles, c'est-à-dire des polymères ayant une solubilité dans l'eau mesurée à 25 °C au moins égale à 0,1 g/L (obtention d'une solution macroscopiquement isotrope et transparente, colorée ou non). Cette solubilité est de préférence supérieure ou égale à 1 g/L.

Le film selon l'invention est de préférence un "film anhydre", c'est-à-dire un film comprenant une teneur en eau inférieure à 15 % en poids, de préférence inférieure à 10 % en poids et plus préférentiellement inférieure à 5 % en poids, par rapport au poids total du film. De préférence encore, film selon l'invention est exempt d'eau.

Les polymères pouvant être utilisés pour constituer ces films peuvent être d'origine synthétique ou naturelle, et, le cas échéant, modifiés par réactions chimiques. Ils peuvent être filmogènes ou non filmogènes. Ils sont avantageusement filmogènes.

Ces polymères doivent être physiologiquement acceptables c'est-à-dire compatible avec la peau, les muqueuses, les cheveux et le cuir chevelu.

La composition sous forme de film comprend notamment au moins un polymère filmogène.

Par "polymère filmogène", on entend un polymère apte à former à lui seul ou en présence d'un agent auxiliaire de filmification, un film continu, et de préférence un film dont la cohésion et les propriétés mécaniques sont telles que ledit film peut être isolé d'un support.

Ces polymères sont catalogués sous la rubrique "Film Formers" dans le dictionnaire cosmétique "International Cosmetic Ingrédient Dictionary and Handbook" (Voir par exemple pages 2903 à 2906 de la neuvième édition - 2002).

Les polymères filmogènes peuvent être choisis par exemple parmi :
- les polymères vinyliques tels que l'acétate de polyvinyle, les polyvinylpyrrolidones, les copolymères du méthylvinyléther et de l'anhydride maléique, le copolymère de l'acétate de vinyle et de l'acide crotonique, les copolymères de la vinylpyrrolidone et de l'acétate de vinyle, les copolymères de la vinylpyrrolidone et du caprolactame, les alcools polyvinyliques. De manière préférée, le polymère vinylique est l'acétate de polyvinyle (PVA), qui est notamment préparé par polymérisation radicalaire du monomère acétate de vinyle puis hydrolyse. On peut utiliser notamment l'acétate de polyvinyle hydrolysé à 88 %, tel que celui vendu sous la dénomination CELVOL® 540 PV ALCOHOL par la société Celanese Chemicals ;
- les dérivés cellulosiques filmogènes, comme l'hydroxyéthylcellulose, l'hydroxypropylcellulose, l'hydroxypropylméthylcellulose, la méthylcellulose, l'éthylhydroxyéthylcellulose, la carboxyméthylcellulose, et les dérivés quaternisés de la cellulose. De manière préférée, les dérivés cellulosiques sont choisis parmi l'hydroxypropylcellulose (HPC) et l'hydroxypropylméthylcellulose (HPMC). Ces polymères sont solubles dans l'eau ainsi que dans des solvants organiques. Ceci permet d'accroître le champ de solubilité des films les contenant. Le choix du poids moléculaire de ces polymères cellulosiques doit être fait de manière judicieuse pour augmenter la dissolution des films. Les HPC utilisés de façon préférée sont ceux commercialisées par la société Hercules sous la dénomination - Klucel® MF dont le poids moléculaire est 850 000 (viscosité 4000-6500 mPa à 2 % dans l'eau) - Klucel® EF dont le poids moléculaire est 80 000 (viscosité 300-600 mPa à 10 % dans l'eau). L'HPMC utilisée de façon préférée est l'hydroxypropylméthylcellulose de viscosité 40-60 cps (40-60 mPa.) à 2 % dans l'eau à 20 °C, commercialisée par la société Sigma-Aldrich.

- les amidons et leurs dérivés ;
- les polymères d'origine naturelle, éventuellement modifiés, tels que le pullulane, la pectine, la mannane, les galactomannanes, les glucomannanes et leurs dérivés, la gomme arabique, la gomme de guar, la gomme de xanthane, la gomme de karaya, les alginates, les carraghénanes, les ulvanes et autres colloïdes algaux, l'acide hyaluronique et ses dérivés, la gomme laque, la gomme sandaraque, les dammars, l'élémis, les copals, l'acide désoxyribonucléique, les mucopolysaccharides tels que l'acide hyaluronique, le sulfate de chondroïtine ;
- les polymères dérivant de la chitine ou du chitosane, anioniques, cationiques, amphotères ou non ioniques ;
- les polymères protéiques, tels que les protéines de blé ou de soja ; la kératine et ses dérivés, par exemple les hydrolysats de kératine et les kératines sulfoniques, la caséine, l'albumine, le collagène, la glutéline, le glucagon, le gluten, la zéine, les gélatines et leurs dérivés ;
- les copolymères acryliques de phosphoryle choline, tels que le poly-2-(méthacryloyloxyéthyl) phosphorylcholine commercialisé sous la dénomination Lipidure® HM par la société NOF Corporation (nom INCI : Polyphosphorylcholine glycol acrylate) ;
- les complexes anion-cation de type gomme arabique / gélatine ou gomme arabique / chitosane, ou collagène / GlycosAminoGlycane ;
et les mélanges de ces polymères.

Selon un mode préféré de réalisation de l'invention, le polymère filmogène est choisi parmi les polymères vinyliques, les dérivés cellulosiques et leurs mélanges.

De manière préférée, le polymère filmogène est choisi parmi l'acétate de polyvinyle, l'hydroxypropylcellulose, l'hydroxypropylméthylcellulose, et leurs mélanges, les polyuréthanes, les nitrocelluloses, et les carbomers.

En particulier, la composition selon l'invention comprend un ou plusieurs polymère(s) filmogène(s) en une teneur allant de 10 à 95 % en poids, de préférence de 20 à 70 % en poids, et plus préférentiellement de 30 à 60% en poids, par rapport total du film.

Lorsqu'elle se présente sous forme de film anhydre, la composition selon l'invention peut comprendre au moins un solvant organique qui peut être choisi parmi :
- les cétones telles que la méthyléthylcétone, la méthylisobutylcétone, la diisobutylcétone, l'isophorone, la cyclohexanone, l'acétone;
- les alcools liquides à température ambiante tels que l'éthanol, l'isopropanol, le diacétone alcool, le 2-butoxyéthanol, le cyclohexanol, le n-propanol, le n-butanol ; les éthers de propylène glycol liquides à température ambiante tels que le monométhyléther de propylène glycol, l'acétate de monométhyl éther de propylène glycol, le mono n-butyl éther de dipropylène glycol ;
- les glycols tels que l'éthylène glycol, le propylène glycol, le pentylène glycol, le glycérol
- les éthers cycliques tels que la γ-butyrolactone ;
- les esters à chaîne courte (ayant de 3 à 8 atomes de carbone au total) tels l'acétate d'éthyle, l'acétate de butyle, l'acétate de méthyle, l'acétate de propyle, l'acétate d'isopropyle, l'acétate d'isopentyle, l'acétate de méthoxypropyle, le lactate de butyle ;
- les éthers tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde;
- le 3-éthoxypropionate d'éthyle ;
- les carbonates tels que le carbonate de propylène, le carbonate de diméthyle,
- les acétals tels que le méthylal ;
et leurs mélanges.

Selon un mode préféré de réalisation, le solvant organique est volatil.

Par " solvant organique volatil", on entend un solvant organique susceptible de s'évaporer au contact de la peau en moins d'une heure, à température ambiante et pression atmosphérique. Le solvant organique volatil est liquide à température ambiante, présente notamment une pression de vapeur non nulle à température ambiante et pression atmosphérique, en particulier il présente une pression de vapeur allant de 0,13 Pa à 40 000 Pa (10<-3> à 300 mm de Hg), et de préférence allant de 1,3 Pa à 8 000 Pa (0,01 à 60 mm de Hg).

Selon un mode particulièrement préféré de réalisation, le solvant organique est choisi parmi les alcools liquides à température ambiante tels que l'éthanol, l'isopropanol, le diacétone alcool, le 2-butoxyéthanol, le cyclohexanol et leurs mélanges, et de préférence l'éthanol et/ou les esters à chaîne courte (ayant de 3 à 8 atomes de carbone au total) tels l'acétate d'éthyle, l'acétate de butyle, l'acétate de méthyle, l'acétate de propyle, l'acétate d'isopropyle, l'acétate d'isopentyle, l'acétate de méthoxypropyle, le lactate de butyle.

Selon un mode plus préféré de réalisation, le solvant organique utilisé dans les compositions selon l'invention est choisi parmi l'éthanol, l'acétate d'éthyle ou un mélange de ceux-ci.

Le solvant organique peut représenter de 60 à 95% en poids, de préférence de 65 à 85% en poids, et de préférence de 65 à 80% en poids, par rapport au poids total de la composition.

On peut également utiliser dans la composition de l'invention, un polymère qui soit à la fois un polymère filmogène et un polymère épaississant, choisi par exemple parmi les dérivés cellulosiques et les polymères d'origine naturelle qui peuvent être à la fois filmogènes et épaississants.

Selon un mode de réalisation particulier, la composition selon l'invention, lorsqu'elle se présente sous la forme d'un film, comprend, outre le polymère filmogène, au moins un agent épaississant polysaccharidique.

Les agents épaississants polysaccharidiques utilisés dans le film selon l'invention peuvent être choisis parmi les polysaccharides à pouvoir gélifiant.

On définit par "pouvoir gélifiant" le fait qu'à une concentration supérieure ou égale à 0,5 % en poids dans l'eau, la viscosité des solutions ainsi obtenues est supérieure ou égale à 0,01 Pa.s pour un taux de cisaillement égal à 1, les mesures étant réalisées à 25 °C à l'aide d'un rhéomètre RheoStress R5150 de Haake en configuration cône - plan, les mensurations du cône de mesure étant les suivantes : diamètre : 60 mm et angle : 2°.

Les agents épaississants polysaccharidiques peuvent être choisis notamment parmi la gomme arabique, la gomme de ghatti, la gomme de karaya, la gomme de caroube, la gomme de guar, la gomme de tamarin, la gomme de xanthane, la gellane, les pectines, le tragacanth, l'agar, les alginates, le carrageenan, le furcelleran, le konjac et les dérivés de cellulose, et leurs mélanges.

Selon un mode préféré de réalisation de l'invention, les agents épaississants polysaccharidiques sont choisis parmi les carraghénanes qui sont des polysaccharides linéaires extraits de certaines algues rouges de la famille des Rhodophycées. Ils sont constitués de résidus α-1,3 et β-1,4 galactoses en alternance, de nombreux résidus galactoses pouvant être sulfatés.

Il existe trois types de carraghénanes, appelés Kappa-carraghénane, Iota-carraghénane et Lambda-carraghénane. Cette famille de polysaccharides est décrite par exemple dans le chapitre 3 du livre " Food Gels " édité par Peter HARRIS, Elsevier 1989. On peut en particulier utiliser le carraghénane vendu sous la dénomination SATIAGUM® UTC 10 par la société Degussa.

La quantité d'agent(s) épaississant(s) dans la composition selon l'invention peut aller par exemple de 0,5 à 40 % en poids, en particulier de 1 à 20 % en poids, et plus particulièrement de 5 à 10 % en poids par rapport à son poids total.

En outre, selon un mode de réalisation avantageux, la composition se présentant sous la forme d'un film peut incorporer, en association avec le polymère tel que défini ci-dessus, au moins un dérivé polydiméthylsiloxane oxyalkyléné.

Comme décrit dans le document FR0653343 un tel dérivé a pour intérêt d'accroître significativement la cinétique de dissolution du film hydrosoluble l'incorporant.

Les polydiméthylsiloxanes (PDMS) oxyalkylénés utilisés selon l'invention sont hydrosolubles ou hydrodispersibles.

On entend par " hydrosolubles ou hydrodispersibles " des PDMS ayant une solubilité dans l'eau mesurée à 25 °C au moins égale à 0,1 g/L (obtention d'une solution macroscopiquement isotrope et transparente, colorée ou non). Cette solubilité est de préférence supérieure ou égale à 1 g/l.

Ces PDMS sont choisis de préférence parmi les silicones hydrosolubles comportant au moins un groupement polyoxyalkyléné monovalent terminal ou pendant, et qui, introduits à 0,05 % en poids dans une solution aqueuse, sont susceptibles de réduire la tension superficielle de l'eau à une valeur inférieure à 35 mN/m, et de préférence inférieure à 30 mN/m.

De tels PDMS oxyalkylénés sont par exemple vendus par la société OSI sous les dénominations commerciales Silwet, Silwet 1614, et Tegowet.

Selon un mode particulièrement préféré de réalisation, la composition selon l'invention se présente sous la forme d'un film anhydre hydrosoluble comportant (i) au moins un oligosaccharide polysulfaté synthétique, (ii) au moins un polymère filmogène hydrosoluble ou hydrodispersible et (iii) au moins un dérivé polydiméthylsiloxane oxyalkyléné.

La composition selon l'invention, notamment lorsqu'elle se présente sous la forme d'un film, peut comprendre en outre un ou plusieurs plastifiants choisis par exemple parmi l'huile de ricin, ou les polyols tels que la glycérine, le sorbitol, les mono- et/ou di-saccharides, le dipropylène glycol, le butylène glycol, le pentylène glycol, les polyéthylènes glycols tels que le PEG-400.

La quantité de plastifiant(s) peut aller par exemple de 1 à 40 % en poids et mieux de 2 à 15 % en poids par rapport au poids total de la composition.

### Procédé de traitement cosmétique

Selon un autre aspect, l'invention se rapporte à un procédé de traitement cosmétique mettant en oeuvre les compositions selon l'invention.

De préférence, l'invention se rapporte à un procédé de traitement cosmétique des vergetures mettant en oeuvre lesdites compositions.

Par procédé de traitement cosmétique, on entend au sens de la présente invention, un procédé non-thérapeutique, en particulier un procédé dont le bénéfice est essentiellement visuel et esthétique.

Le procédé de traitement cosmétique de l'invention peut être mis en oeuvre notamment en appliquant les compositions cosmétiques et/ou dermatologiques ou associations telles que définies ci-dessus, selon la technique d'utilisation habituelle de ces compositions. Par exemple : applications de crèmes, de gels, film polymérique hydrosoluble de type filmogel®, de sérums, de lotions, de laits de démaquillage ou de compositions après-solaires.

Le procédé cosmétique selon l'invention peut être mis en oeuvre par administration topique, journalière par exemple, de l'association selon l'invention qui peut être par exemple formulée sous forme de gels, lotions, émulsions.

Le procédé selon l'invention peut comprendre une administration unique. Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

### Compositions selon l'invention

Selon un mode préféré de réalisation, la composition selon l'invention se présente sous la forme d'une émulsion huile-dans-eau, de préférence sous forme de crème, comprenant :
- 0,1 à 5% de sel de potassium du sucrose octasulfate KSOS
- 40 à 60% en poids d'eau,
- 20 à 40% en poids d'au moins une huile,
- 1 à 10% en poids d'au moins un tensioactif,
- 0 à 10% en poids d'au moins une cire,
- 0 à 20% en poids d'actifs,
- 0 à 2% en poids de conservateurs,
- 0 à 5% en poids d'épaississants.

Selon un autre mode préféré de réalisation, la composition selon l'invention se présente sous la forme d'une composition filmogel à base aqueuse comprenant :
- 0,1 à 5% de sel de potassium du sucrose octasulfate KSOS
- 70 à 99% en poids d'eau,
- 0 à 10% en poids d'actifs,
- 0 à 2% en poids de conservateurs,
- 0 à 5% en poids d'épaississants.

Selon un encore autre mode préféré de réalisation, la composition selon l'invention se présente sous la forme d'une composition filmogel à base solvant comprenant :
- 0,01 à 5% de sel de potassium du sucrose octasulfate KSOS
- 15 à 50% en poids d'éthanol,
- 40 à 60% d'acétate d'éthyle,
- 5 à 20% en poids de polymère filmogène de type nitrocellulose,
- 5 à 20% en poids de plastifiant, de préférence l'huile de ricin,
- 0 à 2% en poids de conservateurs,
- 0 à 5% en poids d'épaississants.

### Utilisation des compositions de l'invention

L'utilisation conforme à l'invention peut être telle que les compositions ou associations définies ci-dessus sont mises en oeuvre dans une formulation destinée à un usage topique.

En particulier, l'invention a pour objet une composition topique pour traiter les vergetures ou les plaies chez un sujet présentant une prédisposition au développement de vergetures comprenant, dans un support physiologiquement acceptable, au moins une quantité efficace d'au moins un oligosaccharide polysulfaté synthétique ayant 1 à 4 unités oses, ses sels et/ou dérivés.

Selon un autre mode de réalisation, l'invention a pour objet un oligosaccharide polysulfaté synthétique ayant 1 à 4 unités oses, ses sels et/ou dérivés pour traiter ou prévenir traiter les cicatrices vergetures.

Selon encore un autre mode de réalisation, l'invention a pour objet l'utilisation d'un oligosaccharide polysulfaté synthétique ayant 1 à 4 unités oses, ses sels et/ou dérivés dans une composition cosmétique pour traiter les vergetures.

Selon encore un autre mode de réalisation, l'invention a pour objet l'utilisation d'un d'oligosaccharide polysulfaté synthétique ayant 1 à 4 unités oses, ses sels et/ou dérivés pour la fabrication d'une composition cosmétique ou dermatologique destinée à traiter ou prévenir traiter les cicatrices vergetures

Les exemples ci-après sont présentés à titre illustratif et non limitatif du domaine de l'invention.

### EXEMPLES

### Exemple 1

On a préparé une formulation sous forme de crème (émulsion huile-dans-eau) comprenant un oligosaccharide polysulfaté synthétique selon l'invention ayant la composition suivante :

| **Constituants** | % |
|---|---|
| Tensioactif huile-dans-eau | 5,000 |
| Cire émulsionnante | 2,000 |
| Acide stéarique | 1,000 |
| Isononanoate d'isodécyle | 6,000 |
| Huile de silicone (Décaméthyl-Cyclopentasiloxane) | 4,000 |
| Ester émollient (myristyl lactate) | 5,000 |
| Eau déminéralisée | 62,100 |
| Epaississant | 0,300 |
| Glycérine | 5,000 |
| Propylène glycol | 5,000 |
| Sel de potassium du sucrose octasulfate KSOS | 0,500 |
| Conservateur | 1,500 |
| NaOH 10% | 0,600 |
| Surfactant siliconé | 2,000 |

On a dispersé l'épaississant dans l'eau. On a ajouté la glycérine, le propylène glycol, le KSOS et le conservateur et on a homogénéisé. On a chauffé à 70-75°C. Lorsque le mélange atteint 70-75°C, on a ajusté le taux d'eau puis neutralisé avec la soude à 10% et on a ramené la température à 70-75°C.

Dans un même temps, on a mélangé le tensioactif huile-dans-eau, la cire émulsionnante, l'acide stéarique, l'isononanoate d'iodécyle, l'huile de silicone (Décaméthyl-Cyclopentasiloxane), l'ester émollient (myristyl lactate) et on a chauffé à 70-75°C.

Lorsque les 2 mélanges on atteint 70-75°C, on a ajouté le second dans le premier sous vive agitation et on a laissé agiter à chaud pendant 10 minutes.

Puis on a ajouté le surfactant siliconé et on a laissé de nouveau sous agitation à chaud pendant 5 minutes.

Enfin, on a arrêté le chauffage et laissé refroidir à température ambiante, en maintenant une agitation suffisante en fonction de la viscosité du mélange. Le mélange prend un aspect non homogène vers 35°C, mais la crème devient ensuite lisse et brillante.

### Exemple 2

On a préparé une formulation de type filmogel à base solvant comprenant un oligosaccharide polysulfaté synthétique selon l'invention ayant la composition suivante :

| **Constituants** | % |
|---|---|
| Nitrocellulose | 12,800 |
| Huile de ricin | 11,000 |
| Ethanol absolu | 24.90 |
| Acétate d'éthyle | 49.70 |
| Filtres UV | 1,500 |
| Sel de potassium du sucrose octasulfate KSOS | 0,100 |

On a dilué la nitrocellulose dans un mélange acétate d'éthyle éthanol absolu. On a ensuite ajouté l'huile de ricin, les filtres UV et le KSOS jusqu'à dissolution pour obtenir une composition de type filmogel.

### Exemple 3

On a préparé une formulation de type filmogel à base aqueuse comprenant un oligosaccharide polysulfaté synthétique selon l'invention ayant la composition suivante

| **Constituants** | % |
|---|---|
| Eau déminéralisée | 93,200 |
| Epaississant | 0,500 |
| Sorbitol | 2,000 |
| Dextran | 1,000 |
| Sel de potassium du sucrose octasulfate KSOS | 1,000 |
| Méthylparaben | 0,050 |
| Propylparaben | 0,050 |
| Phénoxyéthanol | 0,700 |
| NaOH 10% | 1,500 |

On a dispersé l'épaississant dans l'eau sous vive agitation, puis on a ajouté le sorbitol et le dextran en chauffant à 40°C pour obtenir une meilleure solubilité.

On a ajouté le KSOS, les parabens et le phénoxyéthanol et laissé agiter pour homogénéiser. On a ensuite laissé refroidir jusqu'à température ambiante en arrêtant le chauffage, en ajustant si besoin la perte en eau. Enfin, on a neutralisé avec la soude et laisser agiter 10 minutes avant l'arrêt de l'agitation.

## Revendications

1. Composition cosmétique et/ou dermatologique topique, pour son utilisation pour prévenir et/ou traiter les vergetures, comprenant, dans un support physiologiquement acceptable, au moins une quantité efficace d'au moins un oligosaccharide polysulfaté synthétique ayant 1 à 4 unités oses, ses sels et/ou dérivés.

2. Composition pour son utilisation selon la revendication précédente, **caractérisée en ce qu'**elle comprend au moins un oligosaccharide polysulfaté synthétique ayant 1 ou 2 unités oses choisies de préférence parmi les pentoses et les hexoses, ainsi que les sels et/ou dérivés de ces composés.

3. Composition pour son utilisation selon l'une quelconque des revendications précédentes, **caractérisée en ce que** ledit oligosaccharide polysulfaté synthétique ayant 1 ou 2 unités oses est choisi parmi le sel de potassium du sucrose octasulfaté, le sel d'argent du sucrose octasulfaté, le complexe hydroxyaluminium du sucrose octasulfate et leurs mélanges.

4. Composition pour son utilisation selon l'une quelconque des revendications précédentes, **caractérisée en ce que** ledit oligosaccharide polysulfaté synthétique est présent en une teneur comprise entre 0,1 et 50% en poids, par rapport au poids total de la composition.

5. Composition pour son utilisation selon l'une quelconque des revendications précédentes, **caractérisée en ce que** ledit oligosaccharide polysulfaté synthétique est présent en une teneur telle que la quantité relarguée sur les vergetures est comprise entre 0,001 g/l et 50 g/l, et de préférence encore entre 0,01 et 10 g/l.

6. Composition pour son utilisation selon l'une quelconque des revendications précédentes, **caractérisée en ce qu'**elle comprend une (ou plusieurs) autre(s) substance(s) active(s) choisies parmi les anti-bactériens, les antiseptiques, les antifongiques, les anti-inflammatoires, les actifs favorisant la cicatrisation et/ou la restructuration de la peau, les antiprurigineux, les filtres UV, les agents apaisants, les actifs hydratants, les agents dépigmentants, les agents kératolytiques, les anti-viraux, les anti-douleurs, les anesthésiques, les vitamines, et leurs mélanges.

7. Composition pour son utilisation selon la revendication précédente, **caractérisée en ce que** la substance active est choisie parmi les actifs favorisant la cicatrisation et/ou la restructuration de la peau, et notamment l'acide hyaluronique.

8. Composition pour son utilisation selon l'une quelconque des revendications précédentes, **caractérisée en ce qu'**elle se présente sous la forme de solutions (aqueuses, hydroalcooliques ou huileuses), de dispersions (type lotion ou sérum), d'émulsions de consistance liquide ou semi-liquide (type lait), de suspensions ou émulsions (type crème), de gel aqueux ou lipophile, de microémulsions, de microcapsules, de microparticules, de dispersions vésiculaires de type ionique et/ou non ionique, de pansements interfaces non absorbants.

9. Composition pour son utilisation selon l'une quelconque des revendications 1 à 7, **caractérisée en ce qu'**elle se présente sous la forme d'une émulsion huile-dans-eau, de préférence sous forme de crème, comprenant :
- 0,1 à 5% de sel de potassium du sucrose octasulfate KSOS,
- 40 à 60% en poids d'eau,
- 20 à 40% en poids d'au moins une huile,
- 1 à 10% en poids d'au moins un tensioactif,
- 0 à 10% en poids d'au moins une cire,
- 0 à 20% en poids d'actifs,
- 0 à 2% en poids de conservateurs,
- 0 à 5% en poids d'épaississants.

10. Composition pour son utilisation selon l'une quelconque des revendications précédentes, **caractérisée en ce qu'**elle se présente sous la forme d'un film.

11. Composition pour son utilisation selon la revendication précédente, **caractérisée en ce qu'**elle comprend un ou plusieurs polymère(s) filmogène(s) en une teneur allant de 10 à 95 % en poids, de préférence de 20 à 70 % en poids, et plus préférentiellement de 30 à 60% en poids, par rapport total du film.

12. Composition pour son utilisation selon l'une quelconque des revendications 10 ou 11, **caractérisée en ce qu'**elle comprend en outre un agent épaississant polysaccharidique.

13. Composition pour son utilisation selon l'une quelconque des revendications 10 à 12, **caractérisée en ce qu'**elle se présente sous la forme d'un film anhydre hydrosoluble comportant (i) au moins un oligosaccharide polysulfaté synthétique, (ii) au moins un polymère filmogène hydrosoluble ou hydrodispersible et (iii) au moins un dérivé polydiméthylsiloxane oxyalkyléné.

14. Composition pour son utilisation selon l'une quelconque des revendications 10 à 13, **caractérisée en ce qu'**elle se présente sous la forme d'une composition filmogel à base solvant comprenant :
- 0,01 à 5% de sel de potassium du sucrose octasulfate KSOS
- 15 à 50% en poids d'éthanol,
- 40 à 60% d'acétate d'éthyle,
- 5 à 20% en poids de polymère filmogène de type nitrocellulose,
- 5 à 20% de plastifiant, de préférence l'huile de ricin,
- 0 à 2% en poids de conservateurs,
- 0 à 5% en poids d'épaississants.

15. Composition pour son utilisation selon l'une quelconque des revendications 10 à 13, **caractérisée en ce qu'**elle se présente sous la forme d'une composition filmogel à base aqueuse comprenant :
- 0,1 à 5% de sel de potassium du sucrose octasulfate KSOS,
- 70 à 99% en poids d'eau,
- 0 à 10% en poids d'actifs,
- 0 à 2% en poids de conservateurs,
- 0 à 5% en poids d'épaississants.

16. Composition pour son utilisation selon l'une quelconque des revendications précédentes, pour traiter les vergetures ou les plaies chez un sujet présentant une prédisposition au développement de vergetures.

## Patentansprüche

1. Kosmetische und/oder dermatologische topische Zusammensetzung zur Anwendung in der Prävention und/oder Behandlung von Dehnungsstreifen, welche in einem physiologisch akzeptablen Träger wenigstens eine wirksame Menge wenigstens eines synthetischen polysulfatierten Oligosaccharids mit 1 bis 4 Ose-Einheiten, seiner Salze und/oder Derivate umfasst.

2. Zusammensetzung zur Anwendung nach dem vorangehenden Anspruch, **dadurch gekennzeichnet, dass** sie wenigstens ein synthetisches polysulfatiertes Oligosaccharid mit 1 bis 2 Ose-Einheiten umfasst, welche bevorzugt aus den Pentosen und Hexosen gewählt sind, sowie die Salze und/oder Derivate dieser Bestandteile.

3. Zusammensetzung zur Anwendung nach einem der vorangehenden Ansprüche,
**dadurch gekennzeichnet, dass** das synthetische polysulfatierte Oligosaccharid mit 1 bis 2 Ose-Einheiten gewählt ist aus dem Kaliumsalz des Saccharose-Oktasulfats, dem Silbersalz des Saccharose-Oktasulfats, dem Hydroxyaluminium-Komplex des Saccharose-Oktasulfats und deren Mischungen.

4. Zusammensetzung zur Anwendung nach einem der vorangehenden Ansprüche,
**dadurch gekennzeichnet, dass** das synthetische polysulfatierte Oligosaccharid mit einem Gehalt zwischen 0,1 und 50 Gewichtsprozent in Bezug auf das Gesamtgewicht der Zusammensetzung vorliegt.

5. Zusammensetzung zur Anwendung nach einem der vorangehenden Ansprüche,
**dadurch gekennzeichnet, dass** das synthetische polysulfatierte Oligosaccharid mit einem derartigen Gehalt vorliegt, dass die auf die Dehnungsstreifen aufgebrachte Menge zwischen 0,001 g/l und 50 g/l, und noch bevorzugt zwischen 0,01 und 10 g/l liegt.

6. Zusammensetzung zur Anwendung nach einem der vorangehenden Ansprüche,
**dadurch gekennzeichnet, dass** sie einen (oder mehrere) andere(n) Wirkstoff(e) umfasst,
welche gewählt sind aus antibakteriellen Stoffen, antiseptischen Stoffen, antimykotischen Stoffen, entzündungshemmenden Stoffen, Stoffen, die die Wundheilung und/oder Hautrestrukturierung fördern, juckreizstillenden Stoffen, UV-Filtern, beruhigenden Stoffen, feuchtigkeitsspendenden Stoffen, depigmentierenden Stoffen, keratolytischen Stoffen, antiviralen Stoffen, schmerzlindernden Stoffen, anästhetischen Stoffen, Vitaminen, und deren Mischungen.

7. Zusammensetzung zur Anwendung nach dem vorangehenden Anspruch, **dadurch gekennzeichnet, dass** der Wirkstoff aus denjenigen Wirkstoffen gewählt ist, die die Wundheilung und/oder die Hautrestrukturierung fördern, insbesondere Hyaluronsäure.

8. Zusammensetzung zur Anwendung nach einem der vorangehenden Ansprüche, **dadurch gekennzeichnet, dass** sie in Form von Lösungen (wässrig, hydroalkoholisch oder ölig), Dispersionen (vom Typ Lotion oder Serum), flüssigen oder halbflüssigen Emulsionen (vom Typ Milch), Suspensionen oder Emulsionen (vom Typ Creme), wässrigem oder lipophilem Gel, Mikroemulsionen, Mikrokapseln, Mikropartikeln, vesikulären Dispersionen vom ionischen und/oder nicht-ionischen Typ sowie nicht absorbierenden Wundverbandschichten vorliegt.

9. Zusammensetzung zur Anwendung nach einem der Ansprüche 1 bis 7, **dadurch gekennzeichnet, dass** sie in der Form einer Öl-in-Wasser-Emulsion vorliegt, bevorzugt in Form von Creme, umfassend:
- 0,1 bis 5 Prozent Kaliumsalz des Saccharose-Oktasulfats KSOS
- 40 bis 60 Gewichtsprozent Wasser,
- 20 bis 40 Gewichtsprozent wenigstens eines Öls,
- 1 bis 10 Gewichtsprozent wenigstens eines Tensids,
- 0 bis 10 Gewichtsprozent wenigstens eines Wachses,
- 0 bis 20 Gewichtsprozent Wirkstoffe,
- 0 bis 2 Gewichtsprozent Konservierungsmittel,
- 0 bis 5 Gewichtsprozent Dickungsmittel.

10. Zusammensetzung zur Anwendung nach einem der vorangehenden Ansprüche, **dadurch gekennzeichnet, dass** sie als Film vorliegt.

11. Zusammensetzung zur Anwendung nach dem vorangehenden Anspruch, **dadurch gekennzeichnet, dass** sie ein oder mehrere filmbildende Polymere mit einem Gehalt von 10 bis 95 Gewichtsprozent umfasst, bevorzugt von 20 bis 70 Gewichtsprozent und stärker bevorzugt von 30 bis 60 Gewichtsprozent im Verhältnis zum Gesamtfilm.

12. Zusammensetzung zur Anwendung nach einem der Ansprüche 10 oder 11, **dadurch gekennzeichnet, dass** sie ferner ein Polysaccharid-Dickungsmittel umfasst.

13. Zusammensetzung zur Anwendung nach einem der Ansprüche 10 bis 12, **dadurch gekennzeichnet, dass** sie in Form eines wasserlöslichen wasserfreien Films vorliegt, der (i) wenigstens ein synthetisches polysulfatiertes Oligosaccharid, (ii) wenigstens ein filmbildendes wasserlösliches oder wasserdispergierbares Polymer und (iii) wenigstens ein alkoxyliertes Polydimethylsiloxan-Derivat umfasst.

14. Zusammensetzung zur Anwendung nach einem der Ansprüche 10 bis 13, **dadurch gekennzeichnet, dass** sie in Form einer Filmogel-Zusammensetzung auf Lösungsmittelbasis vorliegt, umfassend:
- 0,01 bis 5 Prozent Kaliumsalz des Saccharose-Oktasulfats KSOS,
- 15 bis 50 Gewichtsprozent Ethanol,
- 40 bis 60 Gewichtsprozent Ethylacetat,
- 5 bis 20 Gewichtsprozent filmbildendes Polymer vom Typ Nitrocellulose,
- 5 bis 20 Gewichtsprozent Weichmacher, bevorzugt Rizinusöl,
- 0 bis 2 Gewichtsprozent Konservierungsmittel,
- 0 bis 5 Gewichtsprozent Dickungsmittel.

15. Zusammensetzung zur Anwendung nach einem der Ansprüche 10 bis 13, **dadurch gekennzeichnet, dass** sie in Form einer Filmogel-Zusammensetzung auf wässriger Basis vorliegt, umfassend:
- 0,1 bis 5 Prozent Kaliumsalz des Saccharose-Oktasulfats KSOS
- 70 bis 99 Gewichtsprozent Wasser,
- 0 bis 10 Gewichtsprozent Wirkstoffe,
- 0 bis 2 Gewichtsprozent Konservierungsmittel,
- 0 bis 5 Gewichtsprozent Dickungsmittel.

16. Zusammensetzung zur Anwendung nach einem der vorangehenden Ansprüche, zur Behandlung von Dehnungsstreifen oder Wunden bei einem Patienten mit einer Prädisposition für die Bildung von Dehnungsstreifen.

## Claims

1. A cosmetic and/or dermatological topical composition, for use for preventing and/or treating stretch marks, comprising, in a physiologically acceptable vehicle, at least an effective amount of at least one synthetic polysulfated oligosaccharide having 1 to 4 monosaccharide units, salts and/or derivatives thereof.

2. The composition for use according to the preceding claim, **characterized in that** it comprises at least one synthetic polysulfated oligosaccharide having 1 or 2 monosaccharide units preferably selected from pentoses and hexoses, as well as the salts and/or derivatives of these compounds.

3. The composition for use according to any one of the preceding claims, **characterized in that** said synthetic polysulfated oligosaccharide having 1 or 2 monosaccharide units is selected from potassium salt of sucrose octasulfate, silver salt of sucrose octasulfate, the hydroxyaluminum complex of sucrose octasulfate and mixtures thereof.

4. The composition for use according to any one of the preceding claims, **characterized in that** said synthetic polysulfated oligosaccharide is present at a content between 0.1 and 50 wt%, relative to the total weight of the composition.

5. The composition for use according to any one of the preceding claims, **characterized in that** said synthetic polysulfated oligosaccharide is present in a content such that the amount salted-out on the stretch marks is between 0.001 g/l and 50 g/l, and more preferably between 0.01 and 10 g/l.

6. The composition for use according to any one of the preceding claims, **characterized in that** it comprises one (or more) other active substance(s) selected from antibacterials, antiseptics, antifungals, anti-inflammatories, active substances promoting healing and/or restructuring of the skin, antipruritics, UV filters, soothing agents, hydrating agents, depigmenting agents, keratolytic agents, antivirals, analgesics, anesthetics, vitamins, and mixtures thereof.

7. The composition for use according to the preceding claim, **characterized in that** the active substance is selected from the active substances promoting healing and/or restructuring of the skin, and notably hyaluronic acid.

8. The composition for use according to any one of the preceding claims, **characterized in that** it is in the form of solutions (aqueous, aqueous-alcoholic or oily), dispersions (lotion or serum type), emulsions of liquid or semiliquid consistency (milk type), suspensions or emulsions (cream type), aqueous or lipophilic gel, microemulsions, microcapsules, microparticles, vesicular dispersions of the ionic and/or nonionic type, nonabsorbent interface dressings.

9. The composition for use according to any one of claims 1 to 7, **characterized in that** it is in the form of an oil-in-water emulsion, preferably in the form of cream, comprising:
- 0.1 to 5% of potassium salt of sucrose octasulfate KSOS,
- 40 to 60 wt% of water,
- 20 to 40 wt% of at least one oil,
- 1 to 10 wt% of at least one surfactant,
- 0 to 10 wt% of at least one wax,
- 0 to 20 wt% of active substances,
- 0 to 2 wt% of preservatives,
- 0 to 5 wt% of thickeners.

10. The composition for use according to any one of the preceding claims, **characterized in that** it is in the form of a film.

11. The composition for use according to the preceding claim, **characterized in that** it comprises one or more film-forming polymer(s) at a content ranging from 10 to 95 wt%, preferably from 20 to 70 wt%, and more preferably from 30 to 60 wt%, relative to the total weight of the film.

12. The composition for use according to any one of claims 10 or 11, **characterized in that** it further comprises a polysaccharide thickener.

13. The composition for use according to any one of claims 10 to 12, **characterized in that** it is in the form of an anhydrous water-soluble film comprising (i) at least one synthetic polysulfated oligosaccharide, (ii) at least one water-soluble or water-dispersible film-forming polymer and (iii) at least one alkoxylated polydimethylsiloxane derivative.

14. The composition for use according to any one of claims 10 to 13, **characterized in that** it is in the form of a solvent-based filmogel composition comprising:
- 0.01 to 5% of potassium salt of sucrose octasulfate KSOS
- 15 to 50 wt% of ethanol,
- 40 to 60% of ethyl acetate,
- 5 to 20 wt% of film-forming polymer of the nitrocellulose type,
- 5 to 20% of plasticizer, preferably castor oil,
- 0 to 2 wt% of preservatives,
- 0 to 5 wt% of thickeners.

15. The composition for use according to any one of claims 10 to 13, **characterized in that** it is in the form of a water-based filmogel composition comprising:
- 0.1 to 5% of potassium salt of sucrose octasulfate KSOS,
- 70 to 99 wt% of water,
- 0 to 10 wt% of active substances,
- 0 to 2 wt% of preservatives,
- 0 to 5 wt% of thickeners.

16. The composition for use according to any one of the preceding claims, for treating stretch marks or wounds in a subject having a predisposition to the development of stretch marks.
